# EUROPEAN PATENT SPECIFICATION

(11) **EP 3 202 339 B1**
(45) Date of publication and mention of the grant of the patent: **08.06.2022**
(21) Application number: 17155242.5
(22) Date of filing: 08.02.2017
(51) Int. Cl.: A61B 17/16

(54) **IMPLANT SPECIFIC DRILL TOOL**
IMPLANTATSPEZIFISCHES BOHRWERKZEUG
OUTIL DE FORAGE SPÉCIFIQUE POUR IMPLANT

(30) Priority: 08.02.2016 US 201615018812
(43) Date of publication of application: 09.08.2017
(73) Proprietor: Episurf IP-Management AB, 114 49 Stockholm (SE)
(72) Inventor: BAKE, Nina, 181 30 Lidingö (SE); OTERO QUEVEDO, Manuel, 117 27 Stockholm (SE); HERO, Niklas, 564 32 Bankeryd (SE)
(74) Representative: Keijser Bergöö, Malin Katarina

(56) References cited:
- AU-A1- 2012 300 838
- US-A1- 2012 053 588

## Description

### Technical field

The present disclosure relates to design methods for designing orthopedic drill tools used for drilling bone recesses for implants.

### Background

Pain and overuse disorders of the joints in the body is a common problem. The weightbearing and articulating surfaces of the knees, and of other joints, are covered with a layer of soft tissue that typically comprises a significant amount of hyaline cartilage. The friction between the cartilage and the surrounding parts of the joint is very low, which facilitates movement of the joints under high pressure. The cartilage is however prone to damage due to disease, injury or chronic wear. Moreover it does not readily heal after damages, as opposed to other connective tissue, and if healed the durable hyaline cartilage is often replaced by less durable fibrocartilage. This means that damages of the cartilage gradually become worse. Along with injury/disease comes a problem with pain and reduced joint mobility which may result in handicap and loss of function. It is therefore important to have efficient means and methods for repairing damaged cartilage in knee joints.

Cartilage damage in a joint may e.g. be repaired by replacing damaged cartilage with an implant, and thereby repairing the cartilage in the joint instead of replacing the whole joint with a joint prosthesis. During such cartilage repair, it is important that the implant is well fitted in the joint, since it may otherwise start to move around, which will make the repair in the joint not last for long. This is also important in order to prevent damage of adjacent and opposing joint tissue. This method therefore requires high precision tools.

### Prior art

WO2013/030371A9 describes a design method for designing an implant specific drill bit that corresponds to the shape of the implant to be implanted. Document AU 2012 300 838 A1 discloses a design method according to the preamble of claim 1.

### Problems with the prior art

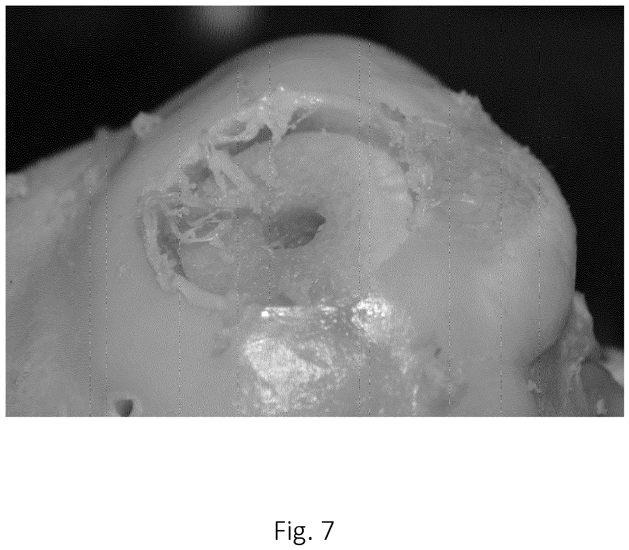

The implant specific drill bit shown in WO2013/030371A9 drills a well-defined recess in the bone that corresponds to the shape of the implant to be implanted for fitting the implant. However, during the drilling of the recess, the tissue above the bone may be damaged, as shown in figure 7. There is therefore a need for an improved drill tool.

### Summary

The above described problems are addressed by the drill tool for implant surgery designed by the design method claimed.

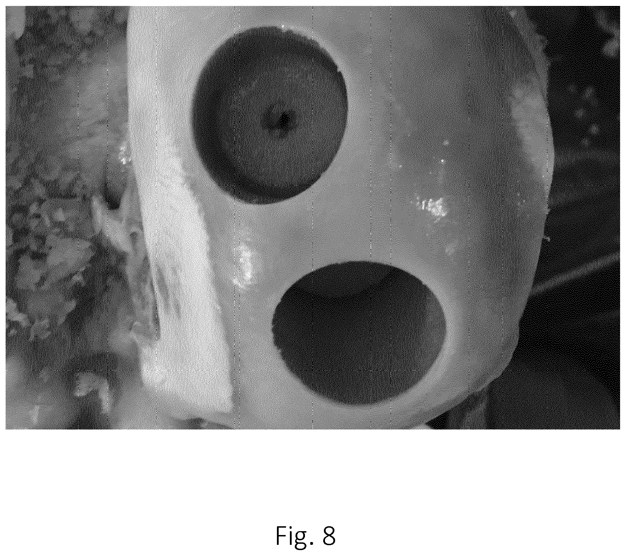

The drill tool comprises a first central drill part having a first diameter for drilling a recess for an implant post, and a second drill part having a second diameter for drilling a recess for an implant hat, the second drill part comprising at least one shape cutting edge arranged peripherally around the first central drill part, and at least one sharp pre-cutting edge extending beyond the at least one shape cutting edge. Thanks to the at least one sharp pre-cutting edge, the cartilage can be cut instead of being mashed by the drill tool (as shown in figure 7), leaving the cartilage edges nice and even (as shown in figure 8).

The at least one sharp pre-cutting edge drills a recess which extends beyond the implant to be implanted.

The at least one shape cutting edge comprises a protruding flange, which provides a recess for an anchoring ring portion protruding from the implant to be implanted. The protruding flange may e.g. have a length of 0.3-3 mm protruding from the shape cutting edge and/or a width 0.3-2.0 mm. The inner side of the protruding flange may e.g. form an angle of less than 20°, or less than 10°, or less than 1° to the longitudinal y-axis of the drill tool.

The above described problems are further addressed by the claimed design method for designing a drill tool for implant surgery for a specific orthopedic implant comprising a circular implant hat and a centrally placed circular extending implant post protruding from the implant hat in a longitudinal y-axis direction of the implant. The drill tool comprises a first central drill part having a first diameter for drilling a recess for the implant post, and a second drill part having a second diameter for drilling a recess for the implant hat. The method comprises:
a) selecting the first diameter of the first central drill part to correspond to, or to be slightly smaller than, the diameter of the implant post;
b) selecting the length of the first central drill part to correspond to, or to be slightly longer than, the length of the implant post;
c) selecting the second diameter of the second drill part to correspond to, or to be slightly smaller than, the diameter of the implant hat;
d) selecting the curvature of one or more shape cutting edges arranged peripherally around the first central drill part to correspond to the curvature of a bone contacting surface of the implant; and
e) adding one or more sharp pre-cutting edges to extend beyond the one or more shape cutting edges and beyond the curvature of the bone contacting surface of the implant.

The at least one shape cutting edge in side view is designed to correspond to the shape of the bone contacting surface of the implant in a cross-sectional view, and the one or more sharp pre-cutting edges extends beyond the bone contacting surface of the implant in a cross-sectional view.

The bone contacting surface is substantially flat and comprises a protruding anchoring ring portion, and the one or more sharp pre-cutting edges is designed to extend beyond the edge of the protruding anchoring ring portion.

In embodiments, the at least one sharp pre-cutting edge is designed to form an angle of less than 60°, or less than 40°, or less than 45° to the longitudinal y-axis of the drill tool.

In embodiments, the first diameter is designed to be 0.1-5% smaller than the diameter of the implant post, and the second diameter is designed to be 0.1-5% smaller than the diameter of the implant hat, allowing for press fit of the implant when placed in the recess made by the drill tool.

In embodiments, the length of the first central drill part is designed to be 1-5 % longer than the length of the implant post.

In embodiments, the first diameter is smaller than the second diameter.

In embodiments, the at least one sharp pre-cutting edge forms an angle of less than 60°, or less than 50°, or less than 45° to the longitudinal y-axis of the drill tool. The pre-cutting edge may have any shape, curvature or angle, as long as it is sharp.

The scope of the invention is defined by the claims, which are incorporated into this section by reference. A more complete understanding of embodiments of the invention will be afforded to those skilled in the art, as well as a realization of additional advantages thereof, by a consideration of the following detailed description of one or more embodiments. Reference will be made to the appended sheets of drawings that will first be described briefly.

### Brief description of the figures

Figure 1 shows a drill tool designed according to an exemplified embodiment of the invention.
Figure 2 shows the lower part of the drill tool shown in figure 1.
Figure 3 schematically illustrates the lower part of a drill tool designed according to another exemplified embodiment of the invention.
Figure 4 shows a schematic embodiment of an implant to be implanted using the drill tool designed according to an exemplified embodiment of the invention.
Figure 5 shows an image of a drill tool designed according to an exemplified embodiment of the invention.
Figure 6 shows a side view image of the drill tool shown in figure 5.
Figure 7 shows an image of a recess drilled in a cartilage coated bone tissue with a conventional drill.
Figure 8 shows an image of two recesses drilled in a cartilage coated bone tissue using one or more drill tools designed according to an embodiment of the invention.

Embodiments of the present disclosure and their advantages are best understood by referring to the detailed description that follows. It should be appreciated that like reference numerals are used to identify like elements illustrated in one or more of the figures.

### Detailed description

The present disclosure relates generally to design methods for designing orthopedic drill tools used for drilling bone recesses for implants. Examples of the drill tool are presented in more detail in connection with the figures.

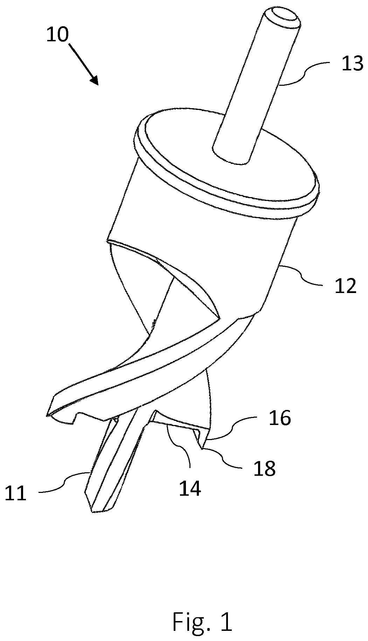

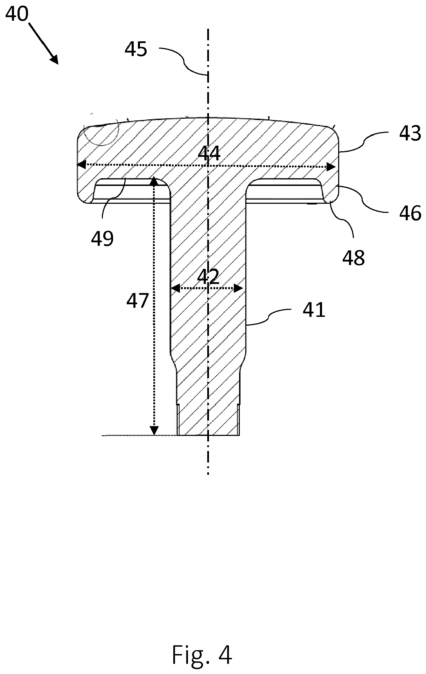

Figure 1 schematically illustrates a drill tool 10 designed according to an exemplified embodiment of the invention. The drill tool 10 is a combination of a drill and bone remover that is used to drill a recess in the bone at the site of cartilage damage, for example in a joint of a patient. The recess is drilled in the same size and shape as the specific implant to be implanted, or slightly smaller than the specific implant to be implanted, and is intended to be used for fastening the implant with a press fit. A suitable implant 40 to be implanted according to the invention is shown in figure 4 and comprises an extending post 41 and an implant hat 43. The implant 40 is to be inserted into the recess formed in the bone tissue and cartilage tissue in the joint using the drill tool 10 according to the invention.

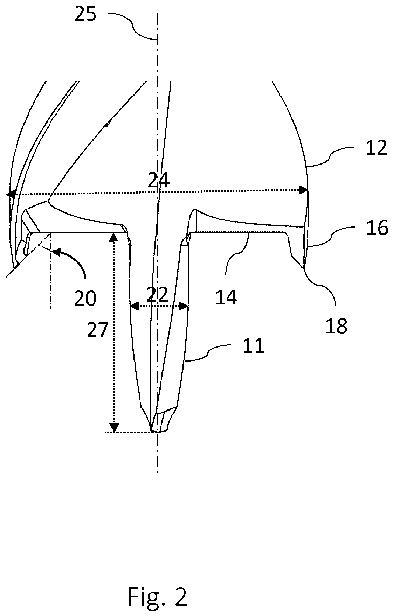

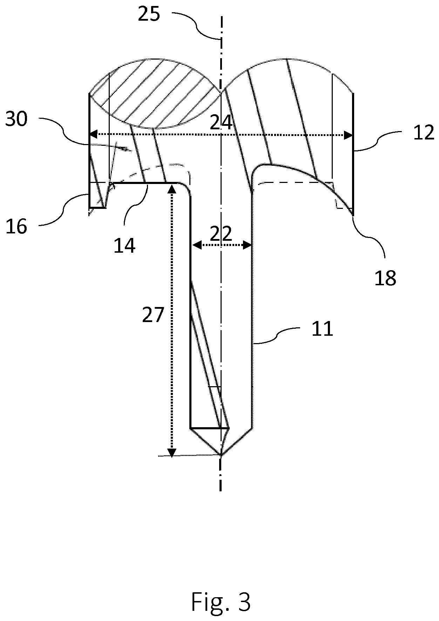

The drill tool 10 comprises a first central drill part 11 and a second drill part 12 which may be a drill and bone remover part, as shown in figure 1. As shown in figures 2 and 3, the first central drill part 11 extends along a longitudinal y- axis 25 of the drill tool 10, i.e. corresponding to the position of the centrally placed extending post 41 on the implant 40 when the drill tool 10 is used for drilling a recess.

The drill tool 10 may further comprise a shaft 13 which may have suitable measures and shapes for use together with a drilling machine. The length of the shaft 13 depends on the need for a long or short shaft for attaching the drill tool 10 to a drilling machine.

Figure 2 shows the lower part of the drill tool 10 shown in figure 1. The drill tool 10 comprises two shape cutting edges 14, each of which comprises a flange 16. The flanges 16 are arranged to drill a recess for a protruding anchoring ring portion or rim 46 on the implant 40. On both flanges 16, sharp pre-cutting edges 18 are arranged. The sharp pre-cutting edges 18 protrude from the flanges 16 in order to cut sharply through the cartilage before drilling the recess in the bone tissue. For examples where the shape cutting edges 14 do not comprise flanges 16, the sharp pre-cutting edges 18 will instead protrude directly from the flat part of the shape cutting edges 14.

Figure 3 schematically illustrates the lower part of a drill tool 10 according to another exemplified embodiment of the invention. The drill tool 10 shown in figure 3 comprises one shape cutting edge 14, which comprises a flange 16. On the opposite side of the drill tool 10, a sharp pre-cutting edge 18 protrudes beyond the shape cutting edge 14 in order to cut sharply through the cartilage before drilling the recess in the bone tissue.

In both of the examples shown in figures 2 and 3, the at least one sharp pre-cutting edge 18 extends beyond the at least one shape cutting edge 14. If the shape cutting edge 14 comprises a flange 16, the at least one sharp pre-cutting edge 18 protrudes beyond the flange 16. The recess drilled by the at least one sharp pre-cutting edge 18 will extend beyond the implant 40 when it is implanted. If the bone contacting surface 49 of the implant 40 comprises a protruding anchoring ring portion 46, the recess drilled by the at least one sharp pre-cutting edge 18 will extend beyond the edge 48 of the protruding anchoring ring portion 46.

The protrusion of the sharp pre-cutting edges 18 beyond the shape cutting edge 14 may e.g. be in the range of 0.1 to 1 mm, such as 0.5 mm. The sharp pre-cutting edge 18 may e.g. form an angle 20 of less than 60°, or less than 50°, or less than 45° to the longitudinal y-axis 25 of the drill tool 10. The sharp pre-cutting edge 18 does not necessarily have the "v"-shape shown in figure 2, but may have any shape or any curvature with any radius, as long as it is sharp.

Different examples may comprise one or more shape cutting edges 14, for example one, two, three or four shape cutting edges. In embodiments, each shape cutting edge 14 is paired with an associated sharp pre-cutting edge 18.

The first central drill part 11 and the shape cutting edge 14 of the second drill part 12 should be hard enough for cutting or carving through bone. The drill tool 10 may e.g. be made of a material such as stainless steel.

Figure 4 shows a schematic example of an implant 40 to be implanted using the drill tool 10 designed to an exemplified embodiment of the invention. The shape and size of the implant are calculated or selected dependent on the size and shape of the cartilage damage, and dependent on the curvature of the contour of the cartilage and/or of the subchondral bone in the area substantially coinciding with the cartilage damage.

The drill tool 10 is implant specific. The diameter 22 of the first central drill part 11 is the same as, or slightly smaller than, the diameter 42 of the extending post 41 of the implant 40 that is selected to be implanted in the joint. The second drill part 12 comprises a shape cutting edge 14 that is placed peripherally around the first central drill part 11. The diameter 24 of the second drill part 12 is the same as, or slightly smaller than, the diameter 44 of the hat 43 of the implant 40 that is to be implanted, thus creating a recess that matches the implant hat 43, in which the implant hat 43 can be received. The shape cutting edge 14 is designed in the same shape as the bone contacting surface 49 of the implant 40 which is selected to be implanted in the bone cavity made using the drill tool 10.

The shape cutting edge 14 may be flat if the implant to be inserted comprises a flat bone contacting surface 49. The shape cutting edge 14 may comprise protruding flanges 16 if the implant hat 43 of the implant 40 to be inserted comprises one or more protruding anchoring ring portions 46. An implant 40 with one or more protruding anchoring ring portions 46 is very well anchored in the bone cavity, since both the extending post 41 and the protruding anchoring ring portion 46 of the implant 40 contributes to the fastening of the implant 40. The drill tool 10 is designed after the shape of the implant 40 to be inserted, but comprises at least one pre-cutting edge 18 which extends beyond the implant 40 to be inserted.

The drill tool 10 is constructed for forming a bone cavity for an implant directly in a joint, and may further be designed to fit the inside of a guide instrument, for example inside a guide channel of a guide body of a guide instrument, with a slight tolerance to allow a sliding movement of the drill tool 10 in such a guide channel. The drill tool 10 may be equipped with a depth gauge and may be used together with a guide tool. The depth gauge of the drill tool 10 determines the depth of the created drill hole as well as the depth of the recess for the implant hat 43. The depth of the drilling may alternatively be adjusted manually, if a drill tool 10 that does not comprise a depth gauge is used, or if the drill tool is used without the guidance of a guide tool.

The drill tool 10 may e.g. have the following measures:
The cross-sectional profile of the drill tool 10 may match or be slightly smaller, for example 0.1-5 volume % smaller than the cross-sectional profile of the implant 40.

The first central drill part 11 may e.g. be cylindrical or conical in shape and have a diameter 22 of 0.7-10 mm (if conical in shape, the diameter refers to the broadest part) and a length 27 of 2-300mm.

The second drill part 12 may e.g. have a diameter 24 of 3-40 mm or 5-40 mm. The diameter 24 of the second drill part 12 may approximately correspond to the diameter 44 of the implant hat 43, or e.g. 1-5 % smaller than the diameter 44 of the implant hat 43. The second drill part 12 may e.g. have a length of 2mm-10cm, or 4mm-5cm, or 4mm-3cm, or a length which provides the drill tool with sufficient support when used together with a guide tool in a guide channel.

The second drill part 12 comprises at least one shape cutting edge 14, and the at least one shape cutting edge 14 may further comprise at least one protruding flange 16 which may protrude from the surface of the at least one shape cutting edge 14 with a length of approximately 0.3-3 mm and a width of 0.3-1.5 mm or 0.3-2 mm. The at least one protruding flange 16 may be made of a material suitable for cutting bone, for example stainless steel, and may be made of the same material as the shape cutting edge 14.

In examples, as illustrated in figure 3, the inner side of the protruding flange 16 forms an angle 30 of less than 20°, or less than 10°, or less than 1° to the longitudinal y-axis 25 of the drill tool 10. The shape of the protruding flange 16 should correspond to the shape of the anchoring ring portion 46 of the implant 40 to be implanted.

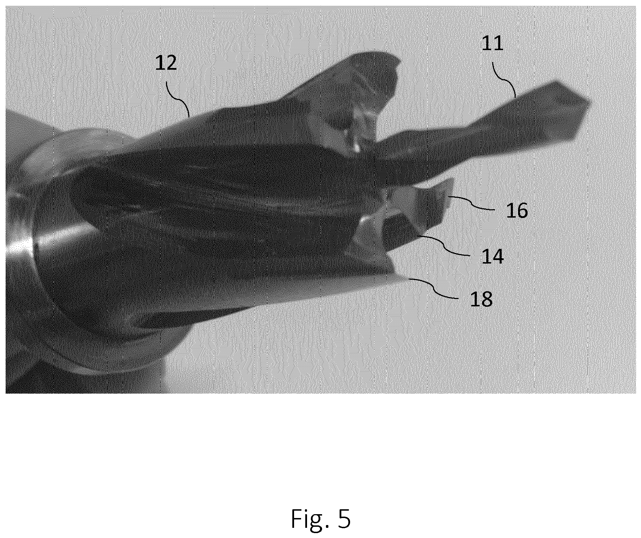

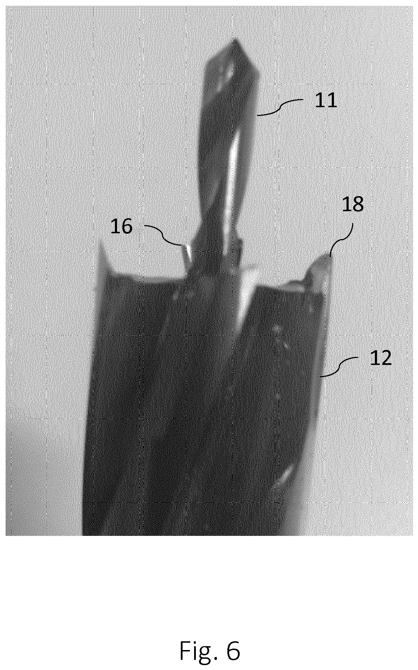

Figures 5 and 6 show images of a drill tool 10 designed according to an exemplified embodiment of the invention. The drill tool 10 comprises two sharp pre-cutting edges 18, and two shape cutting edges 14 comprising flanges 16 without sharp pre-cutting edges 18. The drill tool 10 thus cuts through the cartilage using the sharp pre-cutting edges 18 and then forms the recess in the bone using the two shape cutting edges 14 with the flanges 16. The shape cutting edges 14 with the flanges 16 form the recess to fit the implant 10 to be implanted, while the sharp pre-cutting edges 18 form recesses extending beyond the implant 10 to be implanted.

Figure 7 shows an image of a recess drilled in a cartilage coated bone tissue with a conventional drill, such as the implant specific drill bit described in WO2013/030371A9. It can be seen in the image that the edge of the recess is uneven and the cartilage is frayed. This is because the shape of the drill is adapted entirely to the shape of the recess to be drilled in the bone, and thus does not take into account the cutting of the cartilage. Cartilage is softer than bone, and a drill which is entirely adapted to drill through bone may thus mash the cartilage instead of cutting through it. With conventional technology, the edges of the recess in the cartilage may also be misaligned relative to the edges of the recess of the bone tissue. Figure 8 shows an image of a recess drilled in a cartilage coated bone tissue using a drill tool 10 designed according to an embodiment of the invention. It can be seen in the image that the edge of the recess is even and there is less damage to the surrounding cartilage. The sharp pre-cutting edges 18 cut through the cartilage, leaving the cartilage edges nice and even. The alignment of the recesses in the cartilage and the bone tissue may also be improved because of the sharp pre-cutting edges 18.

The foregoing disclosure is not intended to limit the present invention to the precise forms or particular fields of use disclosed. It is contemplated that various alternate embodiments and/or modifications to the present invention, whether explicitly described or implied herein, are possible in light of the disclosure. Accordingly, the scope of the invention is defined only by the claims.

## Claims

1. A design method for designing a drill tool (10) for implant surgery for a specific orthopedic implant (40) comprising a circular implant hat (43) and a centrally placed circular extending implant post (41) protruding from the implant hat (43) in a longitudinal y-axis (45) direction of the implant (40), the drill tool (10) comprising a first central drill part (11) having a first diameter (22) for drilling a recess for the implant post (41), and a second drill part (12) having a second diameter (24) for drilling a recess for the implant hat (43), the method comprising:
a) selecting the first diameter (22) of the first central drill part (11) to correspond to, or to be slightly smaller than, the diameter (42) of the implant post (41);
b) selecting the length (27) of the first central drill part (11) to correspond to, or to be slightly longer than, the length (47) of the implant post (41);
c) selecting the second diameter (24) of the second drill part (12) to correspond to, or to be slightly smaller than, the diameter (44) of the implant hat (43); and
d) selecting the curvature of one or more shape cutting edges (14) arranged peripherally around the first central drill part (11) to correspond to the curvature of a bone contacting surface (49) of the implant (40);
**characterized in that** the method further comprises:
e) adding one or more sharp pre-cutting edges (18) to extend beyond the one or more shape cutting edges (14) and beyond the curvature of the bone contacting surface (49) of the implant (40), the one or more sharp pre-cutting edges (18) being arranged for cutting sharply through the cartilage, leaving the cartilage edges nice and even, before drilling the recess in the bone tissue, wherein the at least one shape cutting edge (14) in side view is designed to correspond to the shape of the bone contacting surface (49) of the implant (40) in a cross-sectional view, and wherein the one or more sharp pre-cutting edges (18) extends beyond the bone contacting surface (49) of the implant (40) in a cross-sectional view, wherein the bone contacting surface (49) is substantially flat and comprises a protruding anchoring ring portion (46), and the one or more sharp pre-cutting edges (18) is designed to extend beyond the edge (48) of the protruding anchoring ring portion (46).

2. A design method according to claim 1, wherein the first diameter (22) is smaller than the second diameter (24).

3. A design method according to claim 1 or 2, wherein the at least one sharp pre-cutting edge (18) is designed to form an angle (20) of less than 60°, or less than 50°, or less than 45° to the longitudinal y-axis (25) of the drill tool (10).

4. A design method according to any one of claims 1-3, wherein the first diameter (22) is designed to be 0.1-5% smaller than the diameter (42) of the implant post (41), and the second diameter (24) is designed to be 0.1-5% smaller than the diameter (44) of the implant hat (43), allowing for press fit of the implant (40) when placed in the recess made by the drill tool (1).

5. A design method according to any one of claims 1-4, wherein the length (27) of the first central drill part (11) is designed to be 1-5 % longer than the length (47) of the implant post (41).

## Patentansprüche

1. Entwurfsverfahren zum Entwerfen eines Bohrwerkzeugs (10) für Implantatchirurgie für ein spezifisches orthopädisches Implantat (40), das einen kreisförmigen Implantathut (43) und einen mittig angeordneten, sich kreisförmig erstreckenden Implantatpfosten (41) umfasst, der von dem Implantathut (43) in einer längslaufenden y-Achsen- (45) -Richtung des Implantats (40) vorsteht, wobei das Bohrwerkzeug (10) ein erstes mittiges Bohrteil (11) mit einem ersten Durchmesser (22) zum Bohren einer Aussparung für den Implantatpfosten (41) und ein zweites Bohrteil (12) mit einem zweiten Durchmesser (24) zum Bohren einer Aussparung für den Implantathut (43) umfasst, wobei das Verfahren umfasst:
a) Auswählen des ersten Durchmessers (22) des ersten mittigen Bohrteils (11), sodass er dem Durchmesser (42) des Implantatpfostens (41) entspricht oder etwas kleiner als dieser ist;
b) Auswählen der Länge (27) des ersten zentralen Bohrteils (11), sodass sie der Länge (47) des Implantatpfostens (41) entspricht oder etwas länger als dieser ist;
c) Auswählen des zweiten Durchmessers (24) des zweiten Bohrteils (12), sodass er dem Durchmesser (44) des Implantathutes (43) entspricht oder etwas kleiner als dieser ist; und
d) Auswählen der Krümmung einer oder mehrerer Formschneidkanten (14), die umfänglich um das erste mittige Bohrteil (11) herum angeordnet sind, um der Krümmung einer Knochenkontaktfläche (49) des Implantats (40) zu entsprechen;
**dadurch gekennzeichnet, dass** das Verfahren ferner umfasst:
e) Hinzufügen einer oder mehrerer scharfer Vorschneidkanten (18), die sich über die eine oder mehreren Formschneidkanten (14) hinaus und über die Krümmung der Knochenkontaktfläche (49) des Implantats (40) hinaus erstrecken, wobei die eine oder mehreren scharfen Vorschneidkanten (18) so angeordnet sind, dass sie scharf durch den Knorpel schneiden, hinterlassend die Knorpelkanten schön und eben vor Bohren der Aussparung in das Knochengewebe, wobei die wenigstens eine Formschneidkante (14) in Seitenansicht so entworfen ist, dass sie der Form der Knochenkontaktfläche (49) des Implantats (40) in einer Querschnittsansicht entspricht, und wobei sich die eine oder die mehreren scharfen Vorschneidkanten (18) in einer Querschnittsansicht über die Knochenkontaktfläche (49) des Implantats (40) hinaus erstrecken, wobei die Knochenkontaktfläche (49) im Wesentlichen flach ist und einen vorstehenden Verankerungsringabschnitt (46) umfasst und die eine oder die mehreren scharfen Vorschneidkanten (18) so entworfen sind, dass sie sich über die Kante (48) des vorstehenden Verankerungsringabschnitts (46) hinaus erstrecken.

2. Entwurfsverfahren nach Anspruch 1, wobei der erste Durchmesser (22) kleiner ist als der zweite Durchmesser (24).

3. Entwurfsverfahren nach Anspruch 1 oder 2, wobei die wenigstens eine scharfe Vorschneidkante (18) so entworfen ist, dass sie einen Winkel (20) von weniger als 60 °, oder weniger als 50 °, oder weniger als 45 ° zu der längslaufenden y-Achse (25) des Bohrwerkzeugs (10) bildet.

4. Entwurfsverfahren nach einem der Ansprüche 1-3, wobei der erste Durchmesser (22) so entworfen ist, dass er 0,1-5 % kleiner ist als der Durchmesser (42) des Implantatpfostens (41), und der zweite Durchmesser (24) so entworfen ist, dass er 0,1-5 % kleiner ist als der Durchmesser (44) der Implantathuts (43), was Presssitz des Implantats (40) ermöglicht, wenn es in der durch das Bohrwerkzeug (1) gebildete Aussparung angeordnet wird.

5. Entwurfsverfahren nach einem der Ansprüche 1-4, wobei die Länge (27) des ersten mittigen Bohrteils (11) so entworfen ist, dass sie 1-5 % länger ist als die Länge (47) des Implantatpfostens (41).

## Revendications

1. Un procédé de conception pour concevoir un outil de forage (10) pour une chirurgie implantaire pour un implant orthopédique spécifique (40) comprenant un chapeau (43) d'implant circulaire et une tige (41) d'implant à extension circulaire placée au centre, faisant saillie du chapeau d'implant (43) dans une direction longitudinale (45) d'axe y de l'implant (40), l'outil de forage (10) comprenant une première partie centrale de forage (11) ayant un premier diamètre (22) pour forer un évidement pour la tige d'implant (41), et une deuxième partie de forage (42) ayant un deuxième diamètre (24) pour forer un évidement pour le chapeau d'implant (43), le procédé comprenant :
a) le fait de choisir le premier diamètre (22) de la première partie centrale de forage (11) de façon à correspondre au diamètre (42) de la tige d'implant (41) ou à être légèrement inférieur à celui-ci ;
b) le fait de choisir la longueur (27) de la première partie centrale de forage (11) de façon à correspondre à la longueur (47) de la tige d'implant (41), ou être légèrement supérieure à celle-ci ;
c) le fait de choisir le deuxième diamètre (24) de la deuxième partie de forage (12) de façon à correspondre au diamètre (44) du chapeau d'implant (43) ou à être légèrement inférieur à celui-ci ; et
d) le fait de sélectionner la courbure d'une ou plusieurs arêtes tranchantes de coupe (14) agencées de façon périphérique autour de la première partie centrale de forage (11) afin de correspondre à la courbure d'une surface (49) de l'implant (40), de contact avec l'os ;
**caractérisé en ce que** le procédé comprend en outre :
e) le fait d'ajouter une ou plusieurs arêtes tranchantes de pré-coupe (18) de façon à s'étendre au-delà desdites une ou plusieurs arêtes tranchantes de coupe (14) et au-delà de la courbure de la surface (49) de l'implant (40), de contact avec l'os, lesdites une ou plusieurs arêtes tranchantes de pré-coupe (18) étant agencées de façon à couper net à travers le cartilage, laissant les bords du cartilage lisses et réguliers, avant de forer l'évidement dans le tissu osseux, ladite au moins une arête tranchantes de coupe (14) étant conçue, en vue de côté, de façon à correspondre à la forme, en vue en section transversale, de la surface (49) de l'implant (40), de contact avec l'os, et lesdites une ou plusieurs arêtes tranchantes de pré-coupe (18) s'étendant au-delà de la surface (49) de l'implant (40), de contact avec l'os, selon une vue en coupe, la surface (49) de contact avec l'os étant sensiblement plate et comprenant une partie annulaire d'ancrage (46) en saillie, et lesdites une ou plusieurs arêtes tranchants de pré-coupe (18) étant conçues de façon à s'étendre au-delà du bord (48) de la portion annulaire d'ancrage (46) en saillie.

2. Un procédé de conception selon la revendication 1, dans lequel le premier diamètre (22) est inférieur au deuxième diamètre (24).

3. Un procédé de conception selon la revendication 1 ou la revendication 2, dans lequel ladite au moins une arête tranchante de pré-coupe (18) est conçue de façon à former un angle (20) inférieur à 60°, ou inférieur à 50°, ou inférieur à 45°, par rapport à l'axe longitudinal y (25) de l'outil de forage (10).

4. Un procédé de conception selon l'une quelconque des revendications 1 à 3, dans lequel le premier diamètre (22) est conçu de façon à être de 0,1 à 5 % inférieur au diamètre (42) de la tige d'implant (41), et le deuxième diamètre (24) est conçu de façon à être de 0,1 à 5 % inférieur au diamètre (44) du chapeau d'implant (43), permettant un engagement ajusté de l'implant (40) lorsqu'il est placé dans l'évidement réalisé par l'outil de forage (4).

5. Un procédé de conception selon l'une quelconque des revendications 1 à 4, dans lequel la longueur (27) de la première partie centrale du foret (11) est conçue de façon à être de 1 à 5 % plus longue que la longueur (47) de la tige (41) d'implant.
